(19) Europäisches Patentamt
European Patent Office
Office européen des brevets

(11) Veröffentlichungsnummer: **0 011 237**
**A1**

# (12) EUROPÄISCHE PATENTANMELDUNG

(21) Anmeldenummer: **79104382.1**

(22) Anmeldetag: **08.11.79**

(51) Int. Cl.³: **C 08 G 65/32**
A 61 K 31/765

(30) Priorität: **18.11.78 DE 2850058**

(43) Veröffentlichungstag der Anmeldung:
**28.05.80 Patentblatt 80/11**

(84) Benannte Vertragsstaaten:
**AT BE CH DE FR GB IT NL SE**

(71) Anmelder: **BAYER AG**
**Zentralbereich Patente, Marken und Lizenzen**
**D-5090 Leverkusen 1, Bayerwerk(DE)**

(72) Erfinder: **Linke, Siegfried, Dr.**
**Am Hochsitz 23**
**D-5600 Wuppertal 1(DE)**

(72) Erfinder: **Mardin, Mithat, Dr.**
**Bergerheide 39**
**D-5600 Wuppertal 1(DE)**

(72) Erfinder: **Krause, Hans Peter, Dr.**
**Wilkhausstrasse 107**
**D-5600 Wuppertal 2(DE)**

(72) Erfinder: **Sitt, Rüdiger, Dr.**
**Claudiusweg 9**
**D-5600 Wuppertal 1(DE)**

## (54) Polyäther-Derivate, Verfahren zu ihrer Herstellung und ihre Verwendung als Arzneimittel.

(57) Die vorliegende Erfindung betrifft neue Polyäther-Derivate, mehrere Verfahren zu ihrer Herstellung sowie ihre Verwendung als Arzneimittel, insbesondere als Lipidabsorptionshemmer.

EP 0 011 237 A1

0011237

BAYER AKTIENGESELLSCHAFT
Zentralbereich
Patente, Marken und Lizenzen

5090 Leverkusen, Bayerwerk
I b (Pha)
Ks/mo

17. Nov. 1978

Polyäther-Derivate, Verfahren zu ihrer Herstellung und ihre Verwendung als Arzneimittel

Die vorliegende Erfindung betrifft neue Polyäther-Derivate, mehrere Verfahren zu ihrer Herstellung sowie ihre Verwendung als Arzneimittel, insbesondere als Lipidabsorptionshemmer.

Es ist bereits bekannt, daß oberflächenaktive Polyäther, die aus Propylenoxid- und Äthylenoxid-Einheiten bestehen, lipidabsorptionshemmende Eigenschaften besitzen. Von Bochenek und Rodgers wird darauf hingewiesen, daß nichtionische Pluronik-Polyole mit einem hydrophoben Block von 90 % (d.h. 90 % Propylenoxid) lipidabsorptionshemmende Wirkungen aufweisen, während die hydrophileren Polyäther aus der gleichen Reihe die Lipidabsorption nur wenig beeinflussen (vgl. Biochimica et Biophysica Acta, 489, (1977) 503-506).

In dem US-Patent 3 202 578 werden Polyoxyalkylene beschrieben, die als Laxantia Verwendung finden können. In dieser Patentschrift wird neben der laxierenden Wirkung auch eine Wirkung auf die Cholesterinsenkung im Blut erwähnt. Nach den Ausführungen dieser Patentschrift sind solche Polyoxyalkylene besonders als cholesterinsenkende Verbindungen geeignet, die ein Molekulargewicht von ca. 7.500 besitzen und 80 % Äthylenoxid enthalten.

Die Erfindung betrifft Polyäther-Derivate der allgemeinen Formel (I)

$$R^1\text{-}X\text{-}(CH_2\text{-}CH_2\text{-}O)_a\text{-}(\overset{CH_3}{\overset{|}{C}H}\text{-}CH_2\text{-}O)_b\text{-}(CH_2\text{-}CH_2\text{-}O)_c\text{-}CH_2\text{-}CH_2\text{-}X\text{-}R^1$$

(I)

in welcher

X jeweils für Sauerstoff, Schwefel, eine NH- oder eine N-Alkylgruppe steht,

$R^1$ für Alkyl, Alkenyl, Cycloalkyl, Cycloalkenyl, Aryl oder Aralkyl steht, wobei diese Gruppen gegebenenfalls substituiert sind durch Nitro, Cyano, Azido, Halogen, Trifluormethyl, Trifluormethoxy, Phenyl, Hydroxy, Amino, Alkyl, Alkoxy, Alkoxycarbonyl, Acyloxy, Acylamino, Hydroxy, Carboxy oder $SO_2$-Alkyl und

a, b und c für ganze Zahlen stehen, die so gewählt sind, daß ein mittleres Molekulargewicht von 3.000 - 5.000 resultiert und daß der Propylenoxid-Anteil (b) 60 bis 80 % und der Äthylenoxid-Anteil (a und c) 20-40 % beträgt.

Für den Fall, daß der Substituent $R^1$ eine Carboxy- oder Aminogruppe enthält, betrifft die Erfindung auch Salze dieser Verbindungen, die entweder mit Basen oder im Falle der Aminogruppe mit Säuren in an sich bekannter Weise gebildet werden.

Von besonderem Interesse sind Verbindungen der allgemeinen Formel (I) mit einem mittleren Molekulargewicht von ca. 4.000, einem Propylenoxid-Anteil von 70 % und einem Äthylenoxid-Anteil von 30 %.

Überraschenderweise zeigen die Polyäther-Derivate der allgemeinen Formel (I) eine sehr starke lipidabsorptionshemmende Wirkung trotz des niedrigen Molekulargewichtes und einem Äthylenoxid-Anteil von 20 bis 40%.

Die erfindungsgemäßen Polyäther-Derivate der allgemeinen Formel (I) werden erhalten, indem man

a) Polyäther der allgemeinen Formel (II)

$$HO-(CH_2-CH_2-O)_a-(\overset{CH_3}{\overset{|}{C}H}-CH_2-O)_b-(CH_2-CH_2-O)_c-CH_2-CH_2-OH$$

(II)

in welcher

a, b und c die oben angegebene Bedeutung haben,

in einem inerten organischen Lösungsmittel in an sich bekannter Weise mit Natriumhydrid, Natriumamid oder Natriumalkoholat in das entsprechende Dialkoholat überführt und anschließend mit einem Halogenid der allgemeinen Formel (III)

$$Hal\text{-}R^1 \qquad (III)$$

in welcher

$R^1$ die oben angegebene Bedeutung hat und

Hal für Halogen, insbesondere für Brom oder Chlor steht,

umsetzt, oder

b) die Hydroxylgruppen des Polyäthers der allgemeinen Formel (II) nach bekannten Methoden in das Halogenid der allgemeinen Formel (IV)

$$Hal\text{-}(CH_2\text{-}CH_2\text{-}O)_a\text{-}(\underset{|}{\overset{CH_3}{CH}}\text{-}CH_2\text{-}O)_b\text{-}(CH_2\text{-}CH_2\text{-}O)_c\text{-}CH_2\text{-}CH_2\text{-}Hal$$

(IV)

in welcher

a, b, c und Hal die oben angegebene Bedeutung besitzen

und dieses Halogenid dann in Gegenwart eines

inerten Lösungsmittels nach an sich bekannter Weise mit einem Alkohol, Merkaptan oder Amin der allgemeinen Formel (V)

$$R^1\text{-}X\text{-}H \qquad \text{(V)}$$

in welcher

$R^1$ und X die oben angegebene Bedeutung haben,

umsetzt.

Für den Fall, daß $R^1$ Aryl bedeutet und X für Sauerstoff steht, stellt die Verfahrensvariante b) (Umsetzung von IV mit V) eine bevorzugte Ausführungsform dar.

Verwendet man den Polyäther der allgemeinen Formel (II), Natriumhydrid und Bromessigsäure-Äthylester, so kann der Reaktionsablauf gemäß Variante a) durch folgendes Formelschema wiedergegeben werden:

$$HO\text{-}(CH_2\text{-}CH_2\text{-}O)_a\text{-}(\overset{CH_3}{\overset{|}{C}}H\text{-}CH_2\text{-}O)_b\text{-}(CH_2\text{-}CH_2\text{-}O)_c\text{-}CH_2\text{-}CH_2\text{-}OH \xrightarrow[-2H_2]{2NaH}$$

$$Na^{\oplus\ominus}O(CH_2CH_2O)_a(\overset{CH_3}{\overset{|}{C}}HCH_2O)_b(CH_2CH_2O)_cCH_2CH_2O^{\ominus}Na^{\oplus}+2BrCH_2CO_2C_2H_5$$

$$\xrightarrow[-2NaBr]{}$$

$$C_2H_5CO_2CH_2O(CH_2CH_2O)_a(\overset{CH_3}{\overset{|}{C}}HCH_2O)_b(CH_2CH_2O)_cCH_2CH_2OCH_2CO_2C_2H_5$$

Verseifung

$$HOOCCH_2\text{-}O\text{-}(CH_2CH_2O)_a(\overset{CH_3}{\overset{|}{C}}HCH_2O)_b(CH_2CH_2O)_c\text{-}CH_2\text{-}CH_2\text{-}O\text{-}CH_2COOH$$

Verwendet man den Polyäther der allgemeinen Formel (II), Thionylbromid und das Natriumsalz von p-Hydroxybenzoesäureäthylester als Ausgangsstoffe, so kann der Reaktionsablauf nach Variante b) durch folgendes Formelschema wiedergegeben werden:

b) 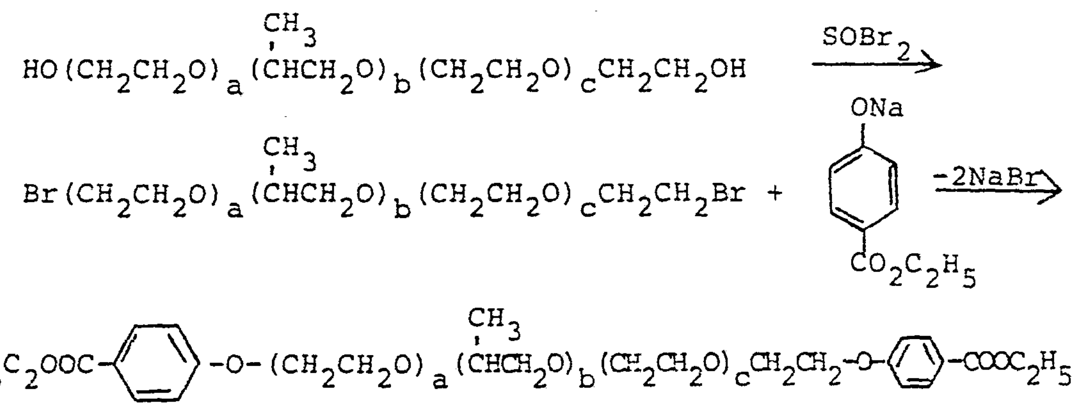


Die als Ausgangsstoffe einzusetzenden Polyäther der allgemeinen Formel (II) sind bekannt oder können nach bekannten Methoden hergestellt werden (vgl. U.S.Pat. 3 674 619 und I.R. Schmolka in J. Am. Oil Chemists Soc. 54, No. 3, 110-16, 1977).

Die als Ausgangsstoffe einzusetzenden Halogenide der allgemeinen Formel (III) sind bekannt oder können nach bekannten Methoden hergestellt werden (vgl. Houben Weyl Bd. 5/3, S. 830-838, 862-870 (1962); Bd. 5/4, S. 361-411, 610-628 (1960).

Als Beispiele seien genannt:

Bromessigsäuremethylester, Bromessigsäureäthylester, 2-Brombuttersäureäthylester, 4-Brombuttersäuremethylester, 4-Bromcrotonsäureäthylester, 2-Bromisobuttersäureäthylester, 2-Brompropionsäureäthylester, 3-Brompropionsäuremethylester, 2-Bromvaleriansäureäthylester, 5-Bromvaleriansäureäthylester.

Von besonderer Bedeutung sind Polyätherderivate der allgemeinen Formel (I)

in welcher

X für Sauerstoff, Schwefel, -NH oder N-Alkyl (1-2 C-Atome) steht und

$R^1$ für Alkyl mit 1 bis 10, insbesondere 1 bis 8 Kohlenstoffatomen, Benzyl oder für einen Phenylrest steht, wobei die Alkylreste gegebenenfalls durch ein oder zwei gegebenenfalls veresterte Carboxylgruppen substituiert sind und wobei der Phenylrest gegebenenfalls substituiert ist durch Alkyl, Alkoxy (je 1-4 C-Atome), Halogen, Nitro oder Trifluormethyl.

Ganz besonders sind Verbindungen der allgemeinen Formel (I)

in welcher

X Sauerstoff bedeutet und

$R^1$ für Alkyl mit 1 bis 4 Kohlenstoffatomen steht, welches durch eine Carbonsäurefunktion substituiert ist.

Die erfindungsgemäßen Verbindungen haben eine enge Molekulargewichtsverteilung.

Die Charakterisierung und Zusammensetzung der erfindungsgemäßen Verbindungen erfolgt analytisch durch Ermittlung des Molekulargewichts aus der Hydroxylzahl. Der Äthylenoxidgehalt wird aus dem $^{1}H$-NMR-Spektrum bestimmt.

**Die erfindungsgemäßen Polyäther zeigen überraschenderweise sehr starke Wirkungen bei der Behandlung von Fett- und Kohlenhydratstoffwechselstörungen. Sie bewirken insbesondere eine Senkung des erhöhten Cholesterins im Serum und im Gewebe und vermindern gleichzeitig eine Hypertriglyceridämie. Die erfindungsgemäßen Verbindungen eignen sich zur Behandlung von Hyperlipoproteinämien, Atherosklerose, Adipositas und zur Behandlung hierdurch ausgelöster Stoffwechselstörungen.**

**Es ist als ausgesprochen überraschend zu bezeichnen, daß die erfindungsgemäßen Polyäther der vorliegenden Anmeldung ausgerechnet in diesem Molekulargewichtsbereich von 3 bis 5000, insbesondere 4000, und dem speziellen Propylenoxid/Äthylenoxid-Verhältnis eine so ausgeprägte hypolipidämische Wirkung besitzen. Da die erfindungsgemäßen Verbindungen neben dieser starken Wirkung gleichzeitig sehr gut verträglich sind, stellen sie eine Bereicherung der Pharmazie dar.**

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, innerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprenmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum-, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Aethylalkohol, Isopropylalkchol, Aethylcarbonat, Aethylacetat, Benzylalkohol, Benzylbenzoat, Propylenglykol, 1,3-Butylenglkyol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Aethylalkohol, Propylenglykol, Suspendiermittel z.B. äthoxylierte Isostearylalkohole, Polyoxyäthylensorbit- und sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B.Pfefferminzöl und Eukalyptusöl, und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung der oben angeführten Erkrankungen.

Die Wirkstoffe oder die pharmazeutischen Zubereitungen können oral, parenteral, intraperitoneal und/oder rektal, vorzugsweise oral, appliziert werden.

Im allgemeinen hat es sich sowohl in der Human- als auch in der Veterinär-Medizin als vorteilhaft erwiesen, den

oder die Wirkstoffe in Mengen von etwa 0,05 bis etwa 500, vorzugsweise 0,5 bis 100 mg/kg Körpergewicht je 24 Stdn., verteilt auf 1 bis 6 Verabreichungen und zwar vor oder/und während oder/und nach der Mahlzeit zu applizieren. Eine Einzelgabe enthält den oder die Wirkstoffe vorzugsweise in Mengen von etwa 0,1 bis etwa 100 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiele:

Beispiel 1 (Verfahrensvariante a)

20 g Polyäther mit dem mittleren Molekulargewicht von 4.000 (Verbindung II) werden in 100 ml Tetrahydrofuran mit 3 g Natriumhydrid unter Stickstoff umgesetzt. Nach 1 Stunde werden 2,6 g Bromessigsäureäthylester zugegeben und anschließend 14 Stunden unter Rückfluß gekocht. Nach Zersetzung des überschüssigen Natriumhydrids wird das Reaktionsgemisch eingedampft und über neutrales Aluminiumoxid mit Chloroform als Laufmittel chromatographiert. Das Eluat wird eingedampft. Der Rückstand zeigt im IR-Spektrum keine OH-Bande aber eine CO-Bande bei 1710 $cm^{-1}$. Nach Verseifen der Estergruppen mit Natronlauge in Äthanol erhält man das erfindungsgemäße Polyätherderivat mit zwei freien Carboxyl-Gruppen. $n_D^{20}$= 1,4572; CO bei 1710 $cm^{-1}$.

Beispiel 2

20 g des im Beispiel 1 eingesetzten Polyäthers werden mit 3 g Natriumhydrid analog Beispiel 1 umgesetzt und anschließend werden 2,2 g Methyliodid zugegeben. Das Reaktionsgemisch wird durch Chromatographie an neutralem Aluminiumoxid gereinigt. Das IR-Spektrum des erhaltenen Dimethyläthers des eingesetzten Polyäthers enthält keine OH-Bande mehr.
$n_D^{20}$ = 1,4561

Beispiel 3

Setzt man anstelle von pro Essigsäureethylester den 3-Brompropionsäureethylester ein, so erhält man analog Beispiel 1 nach Verseifen der beiden Estergruppen das Propionsäurederivat des Polyäthers in öliger Konsistenz mit zwei freien Carboxylgruppen.
$n_D^{20}$ = 1,4570.

Beispiel 4

Verwendet man anstelle von Bromessigsäureethylester den 2-Bromvaleriansäureethylester so erhält man analog Beispiel 1 nach Verseifen der Estergruppen das entsprechende Polyätherderivat in öliger Form.
$n_D^{20}$ = 1,4570.

Beispiel 5

Verwendet man anstelle von Methyljodid das Alkylierungsmittel Ethyljodid so erhält man analog Beispiel 2 das entsprechende Diethylätherderivat des eingesetzten Polyäthers welches keine OH-Bande mehr aufweist in öliger Form.
$n_D^{20}$ = 1,4563.

Beispiel 6

Durch Einsatz von Octyljodid erhält man analog Beispiel 2 den entsprechenden Octyläther in öliger Form.
$n_D^{20}$ = 1,4565.

Beispiel 7

Durch Einsatz von Benzylbromid erhält man analog Beispiel 2 den entsprechenden Benzyläther in öliger Form. $n_D^{20}$ = 1,4569.

Beispiel 8 (Variante b)

80 g Polyäther gemäß Anspruch 1 werden mit 5 ml Thionylbromid umgesetzt. Nach chromatographischer Trennung über Aluminiumoxid erhält man 40 g Polyätherdibromid, welches in 100 ml Äthanol mit 3 g Natriumhydroxid und 2,4 ml Thioessigsäureäthylester versetzt wird. Nach einer Reaktion von 4 Stunden bei 80°C erhält man den entsprechenden Thioäther des Essigsäureäthylesters.

Schwefelgehalt ber. 1,6 %; gef. 1,7 %.
$n_D^{19}$ = 1,4702

Beispiele 9 bis 16

Führt man die Umsetzung analog Beispiel 9 durch und setzt man anstelle von Thioessigsäuremethylester die folgenden Thioalkohole ein behält man nach den analogen Arbeitsbedingungen vom Beispiel 9 die entsprechenden Thioäther des Polyäthers (mittleres MG 4000).

Beispiel 9

Thiophenol: Öl $n_D^{20}$ = 1,4763.

Beispiel 10

4-Chlorthiophenol: Öl $n_D^{20}$ = 1,4765

Beispiel 11

4-tert.-Butylthiophenol:Öl $n_D^{20}$ = 1,4764

Beispiel 12

4-Nitrophenol: Öl $n_D^{20}$ = 1,4765

Beispiel 13

4-Methylthiophenol: Öl $n_D^{20}$ = 1,4767

Beispiel 14

Mercaptoäthanol: Öl $n_D^{20}$ = 1,4666

Beispiel 15

3-Trifluormethylthiophenol: Öl $n_D^{20}$ = 1,4763

Beispiel 16

4-Methoxythiophenol: Öl $n_D^{20}$ = 1,4764

Beispiel 17

36 g Halogenid IV (Hal = Br) werden mit 1 l mit Ammoniak gesättigtem Äthanol versetzt und 7 Tage bei Raumtemperatur stehengelassen. Anschließend wird der Alkohol abgezogen

und das Reaktionsgemisch an neutralem Aluminiumoxid mit Petroläther/Benzol (1:1) chromatographiert.

IR: $-NH_2$ st 3200-3300 $n_D^{20}$ = 1,4605

Stickstoffgehalt ber. 0,7 %

gef. 1,0 %

Beispiele 18 bis 22

Setzt man anstelle von Ammoniak in Beispiel 18 die folgenden Aminverbindungen ein so erhält man nach der Arbeitsweise von Beispiel 18 die entsprechenden Aminderivate des Polyäthers.

Beispiel 18

Dimethylamin: Öl $n_D^{20}$ = 1,4630

Beispiel 19

Methyläthylamin: Öl $n_D^{20}$ = 1,4622

Beispiel 20

N-Methylanilin: Öl $n_D^{20}$ = 1,4635

Beispiel 21

Octylamin: Öl $n_D^{20}$ = 1,4630

Beispiel 22

N-Ethylcyclohexylamin: Öl $n_D^{20}$ = 1,4628

Patentansprüche

1. Polyäther-Derivate der allgemeinen Formel (I)

$$R^1-X-(CH_2-CH_2-O)_a-(\overset{CH_3}{\overset{|}{C}H}-CH_2-O)_b-(CH_2-CH_2-O)_c-CH_2-CH_2-X-R^1$$

(I)

in welcher

X jeweils für Sauerstoff, Schwefel, eine NH- oder N-Alkylgruppe steht,

$R^1$ für Alkyl, Alkenyl, Cycloalkyl, Cycloalkenyl, Aryl oder Aralkyl steht, wobei diese Gruppen gegebenenfalls substituiert sind durch Nitro, Cyano, Azido, Halogen, Trifluormethyl, Trifluormethoxy, Phenyl, Hydroxy, Amino, Alkyl, Alkoxy, Alkoxycarbonyl, Acyloxy, Acylamino, Hydroxy, Carboxy oder $SO_2$-Alkyl und

a,b und c für ganze Zahlen stehen, die so gewählt sind, daß ein mittleres Molekulargewicht von 3.000 - 5.000 resultiert und daß der Propylenoxid-Anteil (b) 60 bis 80 % und der Äthylenoxid-Anteil (a und c) 20-40 % beträgt.

2. Polyätherderivate der allgemeinen Formel (I) gemäß Anspruch 1 in welcher

   a,b,c für ganze Zahlen stehen, die so gewählt sind, daß ein mittleres Molekulargewicht von 4.000 resultiert und daß der Propylenoxid-Anteil (b) 70 % und der Äthylenoxid-Anteil (a und c) 30 % beträgt.

3. Polyätherderivate der allgemeinen Formel (I) gemäß Anspruch 1 in welcher

   X für Sauerstoff, Schwefel, -NH oder N-Alkyl (1 bis 2 C-Atome) steht und

   $R^1$ für Alkyl mit 1 bis 10, insbesondere mit 1 bis 8 Kohlenstoffatomen, Benzyl oder für einen Phenylrest steht, wobei die Alkylreste gegebenenfalls durch 1 oder 2 gegebenenfalls veresterte Carboxylgruppen substituiert sind und wobei der Phenylrest gegebenenfalls substituiert ist durch Alkyl, Alkoxy (mit je 1 bis 4, insbesondere 1 bis 2 Kohlenstoffatomen), Halogen, Nitro oder Trifluormethyl.

4. Verfahren zur Herstellung von Polyäther-Derivaten der allgemeinen Formel (I) gemäß Anspruch 1, dadurch gekennzeichnet, daß man

a) Polyäther der allgemeinen Formel (II)

$$HO-(CH_2-CH_2O)_a-(\overset{CH_3}{\overset{|}{C}H}-CH_2-O)_b-(CH_2-CH_2-O)_c-CH_2-CH_2-OH$$

in welcher

a,b und c die oben angegebene Bedeutung haben,

in einem inerten organischen Lösungsmittel in an sich bekannter Weise mit Natriumhydrid, Natriumamid oder Natriumalkoholat in das entsprechende Dialkoholat überführt und anschließend mit einem Halogenid der allgemeinen Formel (III)

$$Hal-R^1 \qquad (III)$$

in welcher

$R^1$ die oben angegebene Bedeutung hat und

Hal für Halogen, insbesondere für Brom oder Chlor steht,

umsetzt, oder

b) die Hydroxylgruppen des Polyäthers der allgemeinen Formel (II) nach bekannten Methoden in das Halogenid der allgemeinen Formel (IV)

$$Hal-(CH_2-CH_2-O)_a-(\overset{CH_3}{\overset{|}{C}H}-CH_2-O)_b-(CH_2-CH_2-O)_c-CH_2-CH_2-Hal$$

(IV)

in welcher

a, b, c und Hal die oben angegebene Bedeutung besitzen

und dieses Halogenid dann in Gegenwart eines inerten Lösungsmittels nach an sich bekannter Weise mit einem Alkohol, Merkaptan oder Amin der allgemeinen Formel (V)

$$R^1-X-H \qquad (V)$$

in welcher

$R^1$ und X die oben angegebene Bedeutung haben,

umsetzt.

5. Arzneimittel enthaltend mindestens eine Verbindung der allgemeinen Formel (I) gemäß Anspruch 1.

6. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung pharmazeutisch unbedenklicher Hilfs- und Trägerstoffe in eine geeignete Applikationsform überführt.

7. Verwendung von Verbindungen gemäß Anspruch 1 als Lipidadsorbtionshemmer.

8. Behandlung von Erkrankungen des Fettstoffwechsels, dadurch gekennzeichnet, daß man Verbindungen gemäß Anspruch 1 Menschen oder Tieren im Bedarfsfalle appliziert.

Europäisches Patentamt

# EUROPÄISCHER RECHERCHENBERICHT

0011237

Nummer der Anmeldung

EP 79 10 4382

| Kategorie | EINSCHLÄGIGE DOKUMENTE<br>Kennzeichnung des Dokuments mit Angabe, soweit erforderlich, der maßgeblichen Teile | betrifft Anspruch |
| --- | --- | --- |
| X | FR - A - 1 536 689 (BASF)<br>* Zusammenfassung *<br>-- | 1-4 |
| | US - A - 3 957 667 (J. TANIZAKI et al.)<br>* Ansprüche *<br>-- | 1-4 |
| | US - A - 2 662 859 (W.H. KIRKPATRICK)<br>* Anspruch *<br>-- | 1-4 |
| A | BE - A - 691 608 (SMITH KLINE & FRENCH)<br>* Ansprüche *<br>-- | 1,5-8 |
| P | EP - A - 0 000 704 (BAYER)<br>* Ansprüche *<br>---- | 1,5-8 |

KLASSIFIKATION DER ANMELDUNG (Int.Cl.³)

C 08 G 65/32
A 61 K 31/765

RECHERCHIERTE SACHGEBIETE (Int.Cl.³)

C 08 G 65/32
A 61 K 31/74
31/765
31/785
31/795

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
| --- | --- | --- |
| Den Haag | 25-02-1980 | DERAEDT |